# EUROPEAN PATENT SPECIFICATION

(11) **EP 4 378 516 B1**
(45) Date of publication and mention of the grant of the patent: **21.01.2026**
(21) Application number: 22210517.3
(22) Date of filing: 30.11.2022
(51) Int. Cl.: A61M 31/00, A61B 10/00

(54) **INGESTIBLE DEVICE FOR SAMPLING MATERIAL AND DELIVERING DRUG**
EINNEHMBARE VORRICHTUNG ZUR ENTNAHME VON MATERIALPROBEN UND ZUR ABGABE EINES ARZNEIMITTELS
DISPOSITIF INGÉRABLE DE PRÉLÈVEMENT DE MATÉRIAU ET D'ADMINISTRATION DE MÉDICAMENT

(43) Date of publication of application: 05.06.2024
(73) Proprietor: Stichting IMEC Nederland, 6708 WH Wageningen (NL)
(72) Inventor: SCHOEMAN, Rogier, 3001 Leuven (BE)
(74) Representative: Patent Department IMEC

(56) References cited:
- CN-A- 1 820 798
- US-A1- 2008 194 912
- US-A1- 2009 012 503
- US-A1- 2018 070 857
- US-A1- 2020 038 000
- US-A1- 2021 299 419

## Description

### Technical field

The present description relates to an ingestible device for sampling material and delivering drug at least one time and a method for using such an ingestible device.

### Technical background

Generally, to gather information and or samples from the gastrointestinal (GI) tract, endoscopy is often performed. Endoscopes are basically long tubes that can be equipped with a camera, cutting tools, and hollow needles. The proximal and distal parts of the GI tract are reachable via endoscopy. Unfortunately, the endoscopic procedure is rather unpleasant for the patient. On top of that, the small intestine cannot be reached via this procedure. Therefore, the need for a device that enables investigation of the small intestine contents is essential. Moreover, active local drug delivery in the GI tract is challenging at the least.

US 2021/0345904 A1 relates to an ingestible capsule device which collects fluid aspirates from locations within the body, locations such as the small intestine, and retains the fluid aspirates free from contamination as the capsule device is expelled from the body. Said capsule device employs a peristaltic pump fluid control with the capsule device, and a single semi-permeable bladder store collected fluid aspirate. Disadvantageously, especially due to the usage of said peristaltic pump and the rather high energy demand and space requirement associated therewith, said ingestible capsule device is not suitable enough for sensors exemplarily measuring GI fluid in real time.

US 2009/012503 A1 is considered to be the closest prior art to the invention and discloses inter alia an ingestible device for sampling material in a gastrointestinal (GI) tract, the ingestible device comprising: a first chamber being enlargeable in space, comprising an inlet, and to be filled with a material to be sampled; a second chamber comprising a toroidal reservoir being diminishable in space, to be filled with liquid to be delivered; and an actuating mechanism.

### Summary of the invention

Accordingly, there is an object to provide an ingestible device which can provide enough energy and/or free space for sensors measuring GI fluid in real time, especially due to an energy-saving, and/or also space-saving, actuating mechanism. It is also an object to be able to advantageously provide an ingestible device and method for controlled sampling material and delivering a drug.

The scope of the invention is provided by the appended set of claims.

According to a first aspect of the present description, an ingestible device for sampling material in a gastrointestinal (GI) tract is provided. The ingestible device comprises a first chamber being enlargeable in space, comprising an inlet, and to be filled with the material to be sampled, a second chamber being diminishable in space, to be filled with liquid to be delivered; and an actuating mechanism. In this context, the actuating mechanism is configured such that triggering the actuating mechanism leads to deflate the toroidal reservoir so that the first chamber enlarges to collect the material to be sampled through the inlet and the second chamber diminishes to release the liquid through at least one outlet of the toroidal reservoir. The enlargement and the diminishment preferably occur simultaneously. Advantageously, this allows for an energy-saving, and thus also space-saving, actuating mechanism, thereby enabling the provision of enough energy and free space for sensors exemplarily measuring GI fluid in real time.

With respect to the actuating mechanism, it is noted that the actuating mechanism can manually be brought into its inactivated state after it has been activated or after the usage of the ingestible device, respectively. In this context, the actuating mechanism may typically be brought into its inactivated state not in situ.

In addition to this, it is noted that triggering the actuating mechanism may especially comprise actively triggering the actuating mechanism, for example, in a remote location and/or manner.

As a further advantage, it is noted that the ingestible device can be used for applications including human patients as well as animal health.

According to a first preferred example implementation, the first chamber collects the material through the inlet due to an under pressure, wherein the inlet is especially sealed when the material is collected. Additionally or alternatively, the second chamber contains liquid to be released through the at least one outlet due to an over pressure when the second chamber is diminished.

Further additionally or further alternatively, the ingestible device further comprises a third chamber being enlargeable in space, comprising a further inlet, and to be filled with further material to be sampled, a fourth chamber being diminishable in space, comprising at least one further outlet, and containing further liquid to be released, and a further actuating mechanism. In this context, the further actuating mechanism is configured such that triggering the further actuating mechanism leads to deflate the further toroidal reservoir so that the third chamber enlarges to collect the further material through the further inlet due to an under pressure, and to a simultaneous diminishment of the fourth chamber, the fourth chamber thereby releasing the further liquid through the at least one further outlet due to an over pressure. Advantageously, for instance, a second sample can be taken with the same ingestible device especially at a different location in the GI tract.

Further advantageously, it is noted that the ingestible device can and may be used for simultaneously sampling the material and releasing the liquid at least one time.

It might be particularly advantageous if the inlet and the outlet are arranged such that the corresponding physical distance is large enough to exclude taking in material or liquid into the inlet that is released from the outlet.

It is noted that the liquid and/or the further liquid can exemplarily be a drug mixed with the liquid, or an inert liquid, especially in the case if only sampling is desired without a medical treatment of the GI tract. In one example, the liquid and/or the further liquid may comprise drugs that are used against the functional disorders of intestines.

With respect to the further actuating mechanism, it is noted that the further actuating mechanism can manually be brought into its inactivated state after it has been activated or after the usage of the ingestible device, respectively.

It might be particularly advantageous if the further inlet and the at least one further outlet are arranged such that the corresponding physical distance is large enough to exclude taking in material or liquid into the further inlet that is released from the at least one further outlet.

According to a second preferred example implementation, the first chamber and the second chamber are axisymmetric with respect to the third chamber and the fourth chamber. In addition to this or as an alternative, the actuating mechanism is axisymmetric with respect to the further actuating mechanism.

Further advantageously, additionally or alternatively, the first chamber may be mirror-symmetric with the third chamber and/or the second chamber may be mirror-symmetric with the fourth chamber, wherein the respective mirror plane may especially be a cross section in the center of the ingestible device. Additionally or alternatively, the two major segments or halves, respectively, of the ingestible device are basically identical.

According to the invention, the actuating mechanism comprises: a spring being compressed if the actuating mechanism has not been triggered yet, a puncturing element is operatively coupled to the toroidal reservoir. In this context, the puncturing element is configured to deflate the toroidal reservoir if the actuating mechanism is triggered.

Additionally, the spring is arranged with respect to the toroidal reservoir such that deflating the toroidal reservoir leads to a decompression of the spring. Further additionally, the spring is arranged with respect to the first chamber such that the first chamber is enlarged in the case of the decompression of the spring. In further addition to this, the spring is arranged with respect to the second chamber such that the second chamber is diminished in the case of the decompression of the spring. Advantageously, for instance, space can be saved in a particularly efficient manner.

Further advantageously, there is the possibility of using the first chamber and the second chamber similar to a suspension fork principle, i.e., dampening of the spring extension by slow release of liquid from the second chamber through one or more outlets of the toroidal reservoir. In one example, the slow release of liquid from the second chamber may be controlled by precisely controlling the deflation speed of the toroidal reservoir. Accordingly, slow sampling within minutes will reduce the chance of only catching a gas bubble. Also, controlled sampling can be performed by controlling the release of liquid from the second chamber.

According to a further preferred example implementation, the toroidal reservoir and the further toroidal reservoir or the further toroidal reservoir comprises a base part having an opening. Further, an umbrella valve being pressed in the opening of the base part. In addition, a stretchable sheet positioned on top of the base part. In this context, the stretchable sheet is configured to inflate when filled with the liquid through the umbrella valve. Also, the stretchable sheet is configured to deflate when the stretchable sheet is punctured.

Additionally, one or more fastening rings coupled to the base part and the stretchable sheet. In this context, the fastening rings are configured to keep the stretchable sheet intact with the base part so as to provide an airtight seal between the base part and the stretchable sheet. In one embodiment, the stretchable sheet may be glued to the base part to keep the stretchable sheet intact with the base part. Also, in this embodiment, the stretchable sheet may be glued to the base part with or without the fastening rings coupled to the base part.

In one example embodiment, the toroidal reservoir may be molded as a single unitary structure without a base part and/or fastening rings. Also, the toroidal reservoir may be made or molded from a material such as polycaprolactone (PCL). In one example, malleable PCL may be pressed by a mold to form a cavity which is further filled with the liquid. Thereafter, a layer of malleable PCL is placed on top of the cavity, and it is compressed into shape and position to form a filled toroidal reservoir.

According to a further preferred example implementation, the toroidal reservoir is filled with the liquid through the umbrella valve coupled to the inlet of the second chamber or the fourth chamber. In this context, the umbrella valve ceases the filled in liquid and prevents the liquid from flowing out of the toroidal reservoir. In one example, the umbrella valve is a passive one-way valve which allows the liquid to flow into the toroidal reservoir but, ceases or prevents the liquid from flowing out of the toroidal reservoir.

According to a further preferred example implementation, the toroidal reservoir and the further toroidal reservoir may have the shape of a doughnut or a circular tube when it is inflated. Additionally or alternatively, the base part of the toroidal reservoir or the further toroidal reservoir may have an inner diameter in a range from 3 millimeters to 7 millimeters. Further, an outer diameter of the base part is in a range from 7 millimeters to 12 millimeters. Further, the umbrella valve has a diameter in a range from 2.5 millimeters to 6.5 millimeters. In addition, the toroidal reservoir when deflated has a height in a range from 2 millimeters to 3 millimeters. Also, when inflated, the toroidal reservoir has a height in a range from 4 millimeters to 6 millimeters.

According to a further preferred example implementation, the further actuating mechanism comprises a further spring being compressed if the further actuating mechanism has not been triggered yet, a further puncturing element operatively coupled to the toroidal reservoir. In this context, the further puncturing element is configured to deflate the further toroidal reservoir if the further actuating mechanism is triggered. Additionally, the further spring is arranged with respect to the further toroidal reservoir such that deflating the further toroidal reservoir leads to a decompression of the further spring. Also, the further spring is arranged with respect to the third chamber such that the third chamber is enlarged in the case of the decompression of the further spring. In further addition to this, the further spring is arranged with respect to the fourth chamber such that the fourth chamber is diminished in the case of the decompression of the further spring.

Further advantageously, there is the possibility of using the third chamber and the fourth chamber similar to a suspension fork principle, i.e., dampening of the further spring extension by slow release of liquid from the fourth chamber through a small outlet orifice. In one example, the slow release of liquid from the fourth chamber may be controlled by precisely controlling the deflation speed of the further toroidal reservoir. Accordingly, slow sampling within minutes will reduce the chance of only catching a gas bubble. Also, controlled sampling can be performed by controlling the release of liquid from the fourth chamber.

According to a further preferred example implementation, the inlet comprises a valve, preferably a passive valve, more preferably a passive one-way valve, most preferably an umbrella valve. Additionally or alternatively, especially in the case of the presence of the further inlet, the further inlet comprises a further valve, preferably a further passive valve, more preferably a further passive one-way valve, most preferably a further umbrella valve. Advantageously, the umbrella valve and/or the further umbrella valve may be made of silicon rubber.

According to a further preferred example implementation, the first chamber comprises a removable portion, preferably a screwable cap, for access to the internal space of the first chamber. Additionally or alternatively, especially in the case of the presence of the third chamber, the third chamber comprises a further removable portion, preferably a further screwable cap, for access to the internal space of the third chamber. Advantageously, for instance, this allows for an effortless sample retrieval.

According to a further preferred example implementation, the material comprises or is gastrointestinal content. Additionally or alternatively, the liquid comprises or is a drug. Further additionally or further alternatively, especially in the case of the presence of the further material, the further material comprises or is further gastrointestinal content. In further addition to this or as a further alternative, especially in the case of the presence of the further liquid, the further liquid comprises or is a further drug. Advantageously, for example, a simultaneous medical treatment of the GI tract is enabled in a particularly efficient manner.

According to a further preferred example implementation, at least a part of the inner space of the first chamber comprises a stabilizing substance, preferably a quencher, especially for preventing the material from additional chemical reactions. Additionally or alternatively, especially in the case of the presence of the third chamber, at least a part of the inner space of the third chamber comprises a further stabilizing substance, preferably a further quencher, especially for preventing the further material from additional chemical reactions. Advantageously, for instance, digestion and fermentation of the sampled content can be prevented in a particularly efficient manner.

According to a further preferred example implementation, the ingestible device further comprises a body portion especially providing a hollow inner space being preferably sealed against the environment of the body portion exemplarily with the aid of at least one sealing, especially in the form of an O-ring. In this context, the first chamber and the second chamber are attached to the body portion. Additionally or alternatively, especially in the case of the presence of the third chamber and the fourth chamber, the third chamber and the fourth chamber are attached to the body portion preferably in a symmetric manner to the first chamber and the second chamber. Advantageously, for example, sample contamination can be prevented in a cost-efficient manner.

According to a further preferred example implementation, the body portion, especially the hollow inner space of the body portion, comprises at least one of an energy source for supplying electric power to the actuating mechanism and/or in the case of the presence of the further actuating mechanism, for supplying electric power to the further actuating mechanism. Further, the body portion comprises a remote trigger unit, being especially supplied the electric power by the energy source, and configured to receive at least one remote trigger signal in order to trigger the actuating mechanism and/or in the case of the presence of the further actuating mechanism, to trigger the further actuating mechanism. In addition, the body portion comprises a data recording unit for recording data, preferably environment data and/or localization data, during usage of the ingestible device. Furthermore, the body portion comprises a wireless data transmission unit for wirelessly transmitting data, preferably environment data and/or localization data, more preferably environment data and/or localization data in real time, during usage of the ingestible device. Additionally or alternatively, the body portion, especially not the hollow inner space of the body portion, comprises at least one sensor, preferably a pH-value sensor. Advantageously, for instance, there is plenty of room for the energy source, electronics such as communication, preferably wireless communication, and at least one antenna, and sensors.

According to a further preferred example implementation, the ingestible device has the shape of a pill or a cylinder. Additionally or alternatively, the length of the ingestible device is lower than 24 millimeters, especially lower than 23 millimeters, preferably after the actuating mechanism has been triggered and/or in the case of the presence of the further actuating mechanism, the further actuating mechanism has been triggered. Further additionally or further alternatively, the diameter of the ingestible device is lower than 9 millimeters, especially lower than 8 millimeters, preferably after the actuating mechanism has been triggered and/or in the case of the presence of the further actuating mechanism, the further actuating mechanism has been triggered. Advantageously, for example, the ingestible device is small enough in size that it can easily be swallowed.

According to a second aspect of the present description, a method for using an ingestible device according to any of the implementation forms of the first aspect of the invention is provided. The method comprises the step of triggering the actuating mechanism by a first remote trigger signal and/or in the case of the presence of the further actuating mechanism, triggering the further actuating mechanism by transmitting a second remote trigger signal.

### Brief description of the drawings

Exemplary embodiments of the invention are now further explained with respect to the drawings by way of example only, and not for limitation. In the drawings:
- Fig. 1A: shows an example embodiment of an ingestible device according to the present description in an inactivated state;
- Fig. 1B: shows the example embodiment according to Fig. 1A in an activated state;
- Fig. 2A: shows the example embodiment according to Fig. 1A or Fig. 1B, respectively, as a blown-up drawing;
- Fig. 2B: shows the illustration of Fig. 2A in a fully assembled manner and in an inactivated state;
- Fig. 3A: shows an example embodiment of a toroidal reservoir in a deflated state, as a blown-up drawing;
- Fig. 3B: shows an example embodiment of a toroidal reservoir in an inflated state, as a blown-up drawing;
- Fig. 4: shows an example block diagram of a body portion, a puncturing element, a toroidal reservoir, in accordance with aspects of the present description; and
- Fig. 5: shows a flow chart of an example embodiment of a method for using an ingestible device according to embodiments of the present description.

### Detailed description

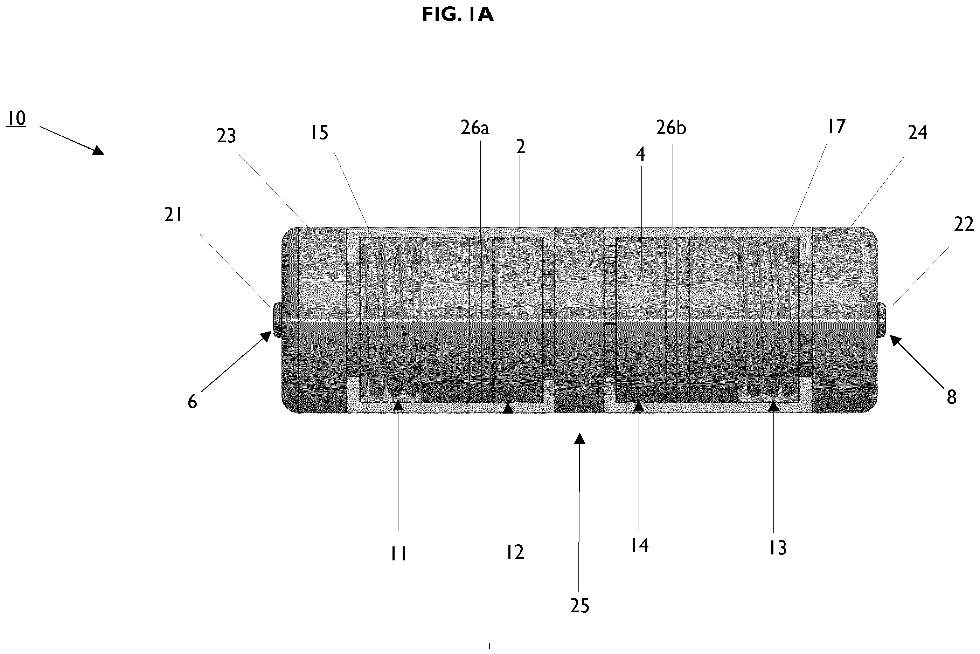

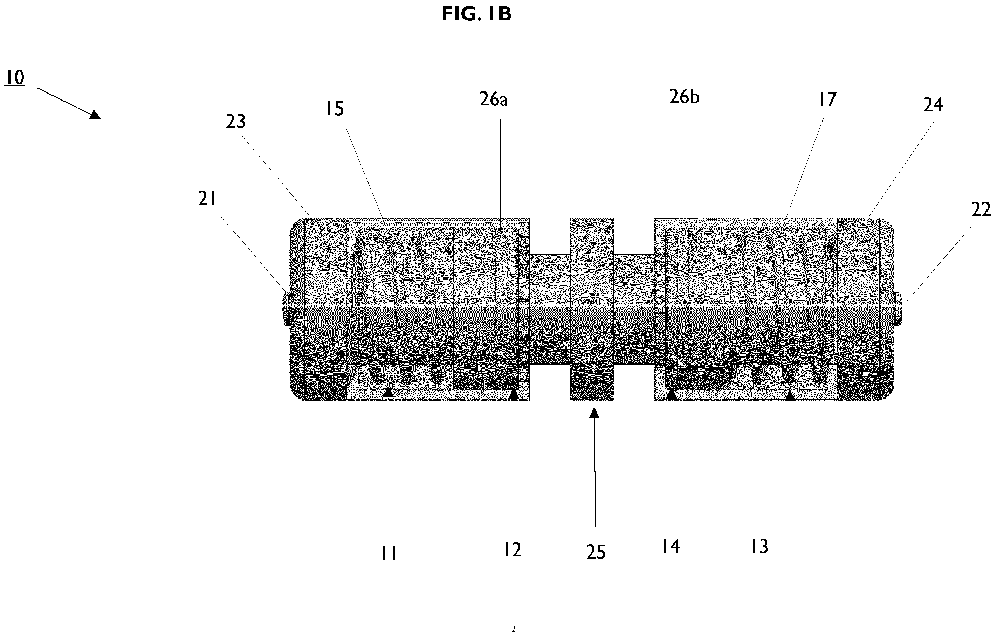

With respect to Figs. 1A and 1B, an exemplary embodiment of an ingestible device 10 for simultaneously sampling material and releasing liquid at least one time is illustrated. Fig. 1A depicts the ingestible device 10 in an inactivated state, while Fig. 1B depicts the ingestible device 10 in an activated state. It may be noted that the inactivated state may be referred to as a state where the actuating mechanism and/or the further actuating mechanism are not triggered, and the toroidal reservoir 2 and/or the further toroidal reservoir 4 are inflated. In one example, the term "inflated" is referred to as filling the toroidal reservoir 2 or the further toroidal reservoir 4 with liquid or drug so that it becomes distended.

According to Fig. 1A, the ingestible device 10 comprises a first chamber 11 being enlargeable in space, comprising an inlet 6, and to be filled with the material to be sampled. In one example, the material may be referred to as gastrointestinal (GI) content that is collected when the ingestible device 10 passes through the GI tract of a body such as, human body or animal body.

Further, the ingestible device 10 comprises a second chamber 12 being diminishable in space, comprising one or more outlets, and containing the liquid to be released. In one example, the liquid may be referred to as one or more drugs either in a liquid state or mixed with other known liquids to be released or delivered within the GI tract when the ingestible device 10 passes through the GI tract of the body. In one example, the drugs may be used against the functional disorders of intestines.

In accordance with Figs. 1A and 1B, the second chamber 12 comprises a toroidal reservoir 2 that is configured to store the liquid when it is inflated and release the stored liquid when it is deflated. Further, it may be noted that the one or more outlets in the second chamber 12 may be referred to as one or more openings occurred in the toroidal reservoir 2 due to puncturing of the toroidal reservoir 2.

In addition, the ingestible device 10 comprises an actuating mechanism. In this context, the actuating mechanism is configured such that triggering the actuating mechanism leads to an enlargement of the first chamber 11 and to a simultaneous diminishment of the second chamber 12. It is noted that the first chamber 11 takes in the material through the inlet 6 especially due to an under pressure, and the second chamber 12 releases the liquid through one or more outlets especially due to an over pressure.

As it can also be seen from Fig. 1A, the ingestible device 10 further comprises a third chamber 13 being enlargeable in space, comprising a further inlet 8, and to be filled with further material to be sampled. The further material may be similar to the above-mentioned material that is referred to as gastrointestinal (GI) content that is collected when the ingestible device 10 passes through the GI tract of the body.

Further, the ingestible device 10 comprises a fourth chamber 14 being diminishable in space, comprising one or more further outlets, and containing further liquid to be released. Also, the further liquid is similar to the above-mentioned liquid that is referred to as one or more drugs mixed with liquids to be released or delivered within the GI tract when the ingestible device 10 passes through the GI tract of the body.

In accordance with Figs. 1A and 1B, the fourth chamber 14 comprises a further toroidal reservoir 4 that is configured to store the liquid when it is inflated and release the stored liquid when it is deflated. Further, it may be noted that the one or more further outlets in the fourth chamber 14 may be referred to as one or more openings that occurred in the further toroidal reservoir 4 due to puncturing of the further toroidal reservoir 4.

In addition, the ingestible device 10 comprises a further actuating mechanism. In this context, the further actuating mechanism is configured such that triggering the further actuating mechanism leads to an enlargement of the third chamber 13, the third chamber 13 thereby taking in further material through the further inlet 8 especially due to an under pressure, and to a simultaneous diminishment of the fourth chamber 14, the fourth chamber 14 thereby releasing the further liquid through the one or more further outlets especially due to an over pressure.

Although Figs 1A and 1B shows two chambers for sampling material and two chambers for delivering the drug, it may be noted that, in one embodiment, the ingestible device 10 may include only one chamber for sampling material and only one chamber for delivering the drug. For example, the ingestible device 10 may include only the first chamber 11 and the second chamber 12, without the third chamber 13 and the fourth chamber 14. Also, the actuating mechanism may be activated/triggered independent of triggering the further actuating mechanism.

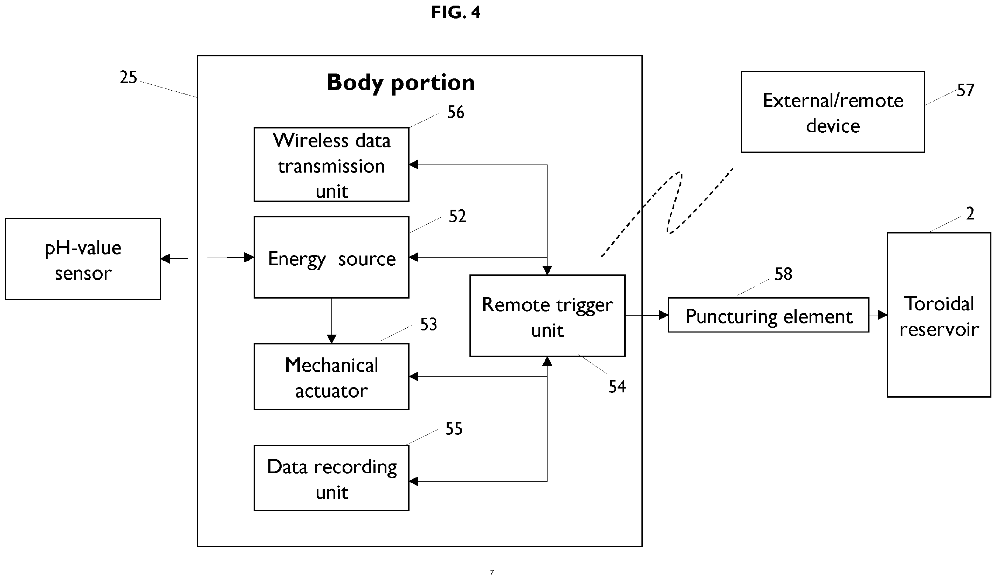

With respect to the above-mentioned actuating mechanism, it is noted that the actuating mechanism comprises a spring 15 and a puncturing element 58 (see Fig. 4). The spring 15 is compressed if the actuating mechanism has not been triggered yet. Further, the puncturing element 58 is operatively coupled to the toroidal reservoir 2. Also, the puncturing element 58 is configured to deflate the toroidal reservoir 2 if the actuating mechanism is triggered. In particular, the puncturing element 58 being conductively connected to an energy source 52 or a mechanical actuator 53 if the actuating mechanism is triggered. This set-up may be seen in Fig. 4. In this context, the puncturing element 58 is configured such that connecting the puncturing element 58 to the energy source 52 leads to puncturing of the toroidal reservoir 2 in the second chamber 12 due to Joule heating.

In a similar manner, if the puncturing element 58 is connected to the mechanical actuator 53, mechanical force is subjected on the puncturing element 58. As a consequence, the puncturing element may melt, poke or tear the toroidal reservoir 2 leading to puncturing of the toroidal reservoir 2 in the second chamber 12. It may be noted that there may be one or more openings on the toroidal reservoir 2 due to puncturing of the toroidal reservoir 2. In one example, the puncturing element 58 may be configured to puncture at one or more locations on the toroidal reservoir 2 so as to control the release of the liquid from the second chamber 12. This in-turn controls the decompression or extension of the spring 15 and the flow of material into the first chamber 11. Also, it may be noted that the toroidal reservoir 2 may also be punctured by other methods and is not limited to heating or poking methods as mentioned above.

In addition to this, the spring 15 is arranged with respect to the toroidal reservoir 2 such that deflating the toroidal reservoir 2 leads to the decompression of the spring 15. As shown in Fig. 1B, the spring 15 is arranged with respect to the first chamber 11 such that the first chamber 11 is enlarged in the case of the decompression of the spring 15. Also, the spring 15 is arranged with respect to the second chamber 12 such that the second chamber 12 is diminished in the case of the decompression of the spring 15.

In accordance with Figs. 1A and 1B, with respect to the further actuating mechanism, it is noted that the further actuating mechanism comprises a further spring 17 and a further puncturing element. It may be noted that the further puncturing element may be similar to the puncturing element 58 shown in Fig. 4. The further spring 17 is compressed if the further actuating mechanism has not been triggered yet.

Further, the further puncturing element is operatively coupled to the further toroidal reservoir 4. Also, the further puncturing element is configured to deflate the toroidal reservoir 4 if the actuating mechanism is triggered. In particular, the further puncturing element, being conductively connected to a further energy source or a further mechanical actuator if the further actuating mechanism is triggered. In this context, the further puncturing element is configured such that connecting the further puncturing element to the further energy source leads to deflating the further toroidal reservoir 4 due to Joule heating.

In a similar manner, if the further puncturing element is connected to the further mechanical actuator, mechanical force is subjected on the further puncturing element which in-turn pokes or tears the further toroidal reservoir 4 leading to puncturing of the further toroidal reservoir 4 in the fourth chamber 14. The further mechanical actuator may push or cause a physical movement of the further puncturing element at a predetermined force towards the further toroidal reservoir to puncture or deflate the further toroidal reservoir. It may be noted that there may be one or more openings on the further toroidal reservoir 4 due to puncturing of the further toroidal reservoir 4. These openings may aid in controlling the release of the liquid from the fourth chamber 14. This in-turn controls the decompression or extension of the further spring 17 and the flow of material into the third chamber 13.

In addition to this, the further spring 17 is arranged with respect to the further toroidal reservoir 4 such that deflating the further toroidal reservoir 4 leads to a decompression of the further spring 17. As shown in Fig. 1B, the further spring 17 is arranged with respect to the third chamber 13 such that the third chamber 13 is enlarged in the case of the decompression of the further spring 17. Also, the further spring 17 is arranged with respect to the fourth chamber 14 such that the fourth chamber 14 is diminished in the case of the decompression of the further spring 17.

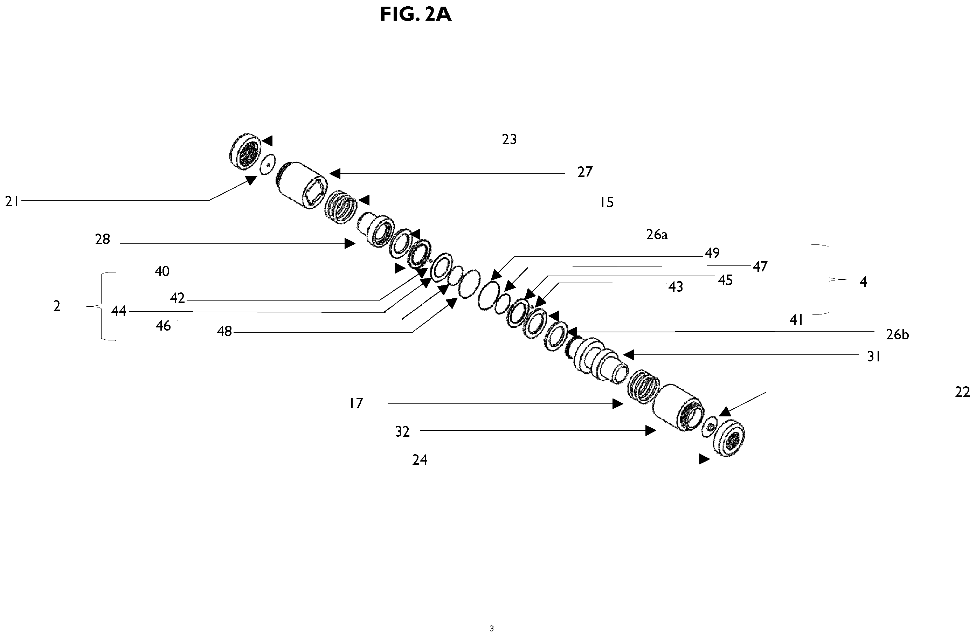

With respect to the above-mentioned inlet 6, it is noted that the inlet 6 comprises a valve, preferably a passive valve, more preferably a passive one-way valve, most preferably an umbrella valve such as the umbrella valve 21 of Fig. 2A. With respect to the above-mentioned further inlet 8, it is noted that the further inlet comprises a further valve, preferably a further passive valve, more preferably a further passive one-way valve, most preferably a further umbrella valve such as the further umbrella valve 22 of Fig. 2A. It may be noted that the valve 21 or the further valve 22 allows flow of material from outside the ingestible device 10 into the chamber (first or third chamber) but, blocks or ceases the material to flow out of the chamber (first chamber or third chamber).

In addition, at least a part of the inner space of the first chamber 11 comprises a stabilizing substance, preferably a quencher, especially for preventing the material from additional chemical reactions. In a similar manner, at least a part of the inner space of the third chamber 14 comprises a further stabilizing substance, preferably a quencher, especially for preventing the further material from additional chemical reactions. Advantageously, for instance, digestion and fermentation of the sampled content can be prevented in a particularly efficient manner by using the stabilizing substance or the further stabilizing substance.

Furthermore, as it can be seen from Figs. 1A and 1B or Fig. 2A, respectively, the first chamber 11 comprises a removable portion, exemplarily a screwable cap 23, for access to the internal space of the first chamber 11. Moreover, the third chamber 13 comprises a further removable portion, exemplarily a further screwable cap 24, for access to the internal space of the third chamber 13.

In accordance with Fig. 1A, the ingestible device 10 further comprises a body portion 25 especially providing a hollow inner space being preferably sealed against the environment of the body portion 25 exemplarily with the aid of two O-rings 26a and 26b. In this context, the first chamber 11 and the second chamber 12 are attached to the body portion 25, and the third chamber 13 and the fourth chamber 14 are attached to the body portion 25 exemplarily in a symmetric manner to the first chamber 11 and the second chamber 12.

It might be particularly advantageous if the body portion 25, especially the hollow inner space of the body portion 25, comprises at least one of an energy source for supplying electric power the actuating mechanism and the further actuating mechanism. Also, the body portion 25 comprises a remote trigger unit, being especially supplied the electric power by the energy source and configured to receive at least one remote trigger signal in order to trigger the actuating mechanism and the further actuating mechanism.

Further, the body portion 25 comprises a data recording unit for recording data, preferably environment data and/or localization data, during usage of the ingestible device 10. In one example, the environment data could be pH and temperature. This data is also used to roughly determine the position of the pill in the GI tract. Other environmental data could be inflammation markers. It may be noted that the body portion 25 is described in greater detail with respect to Fig. 4.

In addition to this or as an alternative, the body portion 25, especially not the hollow inner space of the body portion 25, may comprise at least one sensor, preferably a pH-value sensor. In one example, the pH-value sensor may be used to determine or measure the pH-value at one or more locations in the GI tract. It is further noted that the ingestible device 10 has the shape of a pill or a cylinder.

As it can be seen from Fig. 1A showing the inactivated state of the ingestible device 10 and Fig. 1B illustrating the activated state of the same, the diameter of the ingestible device 10 does not change after activation, whereas the length of the ingestible device 10 increases due to activation. In this context, the length of the ingestible device 10 is lower than 24 millimeters, especially 23 millimeters, exemplarily after the actuating mechanism has been triggered and/or the further actuating mechanism has been triggered. Also, the diameter of the ingestible device 10 is lower than 9 millimeters, especially 8 millimeters.

With respect to the inactivated state of the ingestible device 10 according to Fig. 1A, it is noted that in this state, neither the actuation mechanism nor the further actuation mechanism have been triggered yet. Accordingly, both the spring 15 and the further spring 17 are still compressed. With respect to the activated state of the ingestible device 10 according to Fig. 1B, it is further noted that in this state, at least one, exemplarily both, of the actuation mechanism and the further actuation mechanism has been triggered, which leads to a decompression of the respective one of the springs 15 and the further spring 17.

Again, with respect to Fig. 2A, it is noted that the screwable cap 23, the umbrella valve 21, the spring 15, the O-ring 26a, the further O-ring 26b, the further spring 17, the further umbrella valve 22, the further screwable cap 24, and the body portion 25 have already been explained in the context of Fig. 1A or Fig. 1B, respectively.

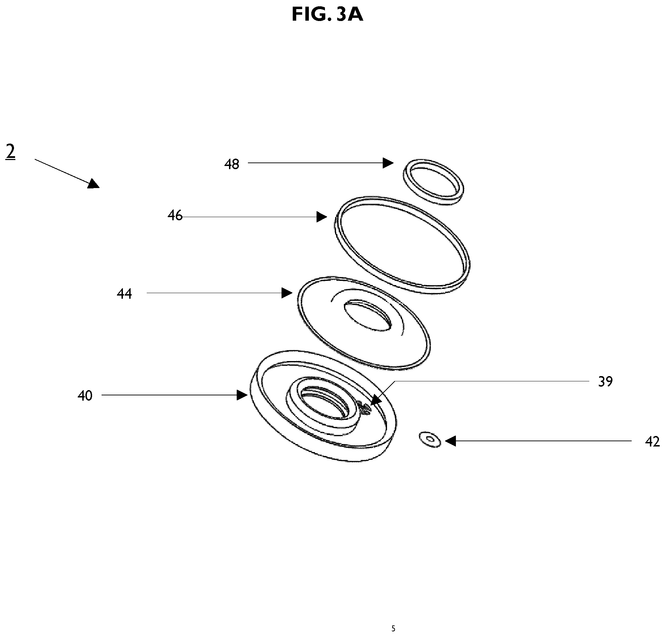

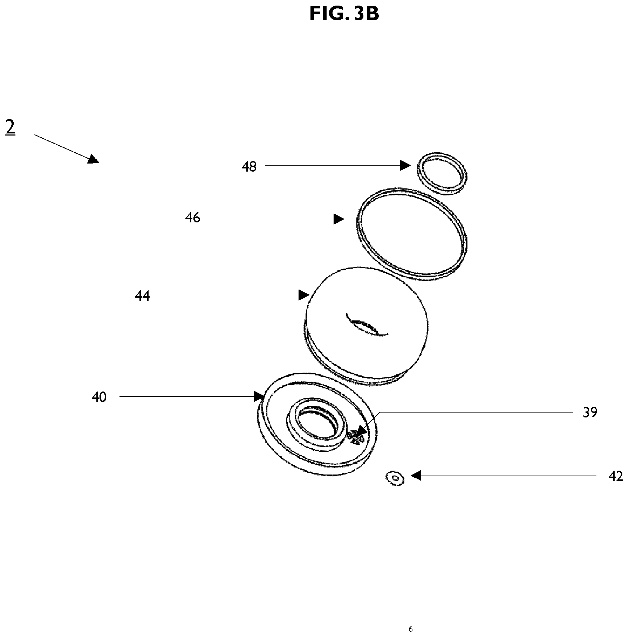

Furthermore, in view of Fig. 2A, it is noted that the toroidal reservoir 2 and the further toroidal reservoir 4 have already been explained in the context of Fig. 1A or Fig. 1B. Furthermore, the internal components such as, a base part 40, 41, umbrella valve 42, 43, stretchable sheet 44, 45, fastening rings 46, 47, 48, 49 of the toroidal reservoir 2 and the further toroidal reservoir 4 will be explained in greater detail with respect to Figs. 3A and 3B.

Fig. 3A shows the blown-up view of the toroidal reservoir that is deflated. Fig. 3B shows the blown-up view of the toroidal reservoir that is inflated. The toroidal reservoir 2 includes a base part 40, an umbrella valve 42, a stretchable sheet 44, one or more fastening rings 46, 48. It may be noted that the further toroidal reservoir 4 is similar to the toroidal reservoir 2, which includes a base part 41, an umbrella valve 43, a stretchable sheet 45, one or more fastening rings 47, 49. For ease of understanding of the invention, the aspect of the toroidal reservoir 2 is explained in greater detail with respect to Fig. 3A.

As depicted in FIG. 3A, the base part 40 includes an opening 39 to which the umbrella valve 42 is pressed and secured. The umbrella valve 42 that is pressed at the opening 39 of the base part 40 may act as an inlet for the liquid to flow through and fill the toroidal reservoir 2. However, the umbrella valve 42 ceases the liquid to flow out of the toroidal reservoir 2.

In addition, the stretchable sheet 44 is position on top of the base part 40 as shown in Fig. 3A. In one example, the stretchable sheet 44 may be made of biocompatible latex such as natural rubber latex or bio-inert such as regular latex. In another example, the stretchable sheet 44 may be of polymer material such as polycaprolactone (PCL). In this context, the stretchable sheet 44 is configured to inflate when it is filled with the liquid through the umbrella valve 42. Further, when the stretchable sheet 44 is punctured, one or more openings are formed on the stretchable sheet 44. Thereafter, the liquid that is filled in the stretchable sheet 44 flows out through these one or more openings on the stretchable sheet 44. It may be noted that the stretchable sheet 44 is punctured when the actuating mechanism is triggered. In particular, the puncturing element 58 (see Fig. 4) is operatively coupled to the stretchable sheet 44 and configured to puncture or deflate the stretchable sheet 44 when the actuating mechanism is triggered. The aspect of triggering and puncturing the toroidal reservoir 2 is explained in greater detail with respect to Fig. 4.

Furthermore, the one or more fastening rings 46, 48 are coupled to the base part 40 and the stretchable sheet 44. In this context the fastening rings 46, 48 are configured to keep the stretchable sheet 44 intact with the base part 40 and to provide an airtight seal between the base part 40 and the stretchable sheet 44. This in-turn prevents leakage of the liquid when the toroidal reservoir is inflated.

As depicted in Fig. 3A, the stretchable sheet 44 is first placed on top of the base part 40. Further, the inner ring 46 and the outer ring 48 are placed on top of the stretchable sheet 44 to tightly couple the stretchable sheet 44 to the base part 40. In one example, the fastening rings 46, 48 may be adhesive rings that helps in providing airtight seal between the base part 40 and the stretchable sheet 44. Fig. 3B shows the blown-up view of the toroidal reservoir that is inflated. It may be noted that Fig. 3B is similar to Fig. 3A except that the stretchable sheet 44 is shown as inflated. Also, it can be seen in Fig. 3B that the stretchable sheet 44 when inflated appears to be in the shape of doughnut or circular tube.

Additionally or alternatively, the base part 40 of the toroidal reservoir 2 or the further toroidal reservoir 4 may have an inner diameter in a range from 3 millimeters to 7 millimeters. Further, an outer diameter of the base part 40 is in a range from 7 millimeters to 12 millimeters. Further, the umbrella valve 42 has a diameter in a range from 2.5 millimeters to 6.5 millimeters. In addition, the toroidal reservoir 2 or the further toroidal reservoir 4 when deflated has a height in a range from 2 millimeters to 3 millimeters. Also, when inflated, the toroidal reservoir 2 or the further toroidal reservoir 4 has a height in a range from 4 millimeters to 6 millimeters.

In one embodiment, the toroidal reservoir 2 may be molded as a single unitary structure without the base part 40 and the fastening rings 46, 48. Also, the toroidal reservoir may be made or molded from a material such as polycaprolactone (PCL).

Moving back to Fig. 2A, the above-mentioned body portion 25 of Fig. 1A or Fig. 1B, respectively, comprises a first body part 28 and a second body part 31. Furthermore, a first outer shell 27 of the ingestible device 10 comprises the first body part 28. Additionally, the ingestible device 10 comprises a second outer shell 32 comprising the second body part 32.

With respect to the body portion 25, it is noted that said body portion, especially its hollow inner space, may comprise one or more electronics modules as depicted in Fig. 4. It might be particularly advantageous if said electronics modules comprises at least an energy source 52, a mechanical actuator 53, a remote trigger unit 54, a data recording unit 55, and a wireless data transmission unit 56. The energy source 52 is configured to supply electric power to the actuating mechanism and the further actuating mechanism. Also, the energy source 52 may supply electric power to the remote trigger unit 54, the data recording unit 55, and the wireless data transmission unit 56, and the mechanical actuator 53. Further, the remote trigger unit 54 is configured to receive at least one remote trigger signal in order to trigger the actuating mechanism and the further actuating mechanism. In one example, the remote trigger unit 54 may receive the remote trigger signal from an external or remote device 57 as shown in Fig. 4.

In this context, if the actuating mechanism is triggered, the energy source 52 or the mechanical actuator 53 is coupled to the puncturing element 58 to puncture or deflate the toroidal reservoir 2. In one example, if the puncturing element 58 is coupled to the energy source 52, the puncturing element 58 heats up or the temperature increases due to joule heating. As a consequence, the puncturing element 58 may deflate or puncture the toroidal reservoir 2, particularly the stretchable sheet 44. In another example, if the puncturing element 58 is coupled to the mechanical actuator 53, mechanical force is created on the puncturing element 58. This in-turn causes physical movement or lateral movement of the puncturing element 58 to poke or tear the toroidal reservoir 2, particularly the stretchable sheet 44 and to deflate the toroidal reservoir.

Furthermore, the data recording unit 55 is configured to record data, preferably environment data and/or localization data, during usage of the ingestible device 10. The wireless data transmission unit 56 is configured to wirelessly transmit data, preferably environment data and/or localization data, more preferably environment data and/or localization data in real time, during usage of the ingestible device 10. In one embodiment, the wireless data transmission unit 56 may also be configured to wirelessly receive the remote trigger signal from the external device 57 and transmit the remote trigger signal to the remote trigger unit 54. In addition, the said electronics module may comprise at least one sensor, preferably a pH-value sensor for sensing pH-value at a desired location in the GI tract.

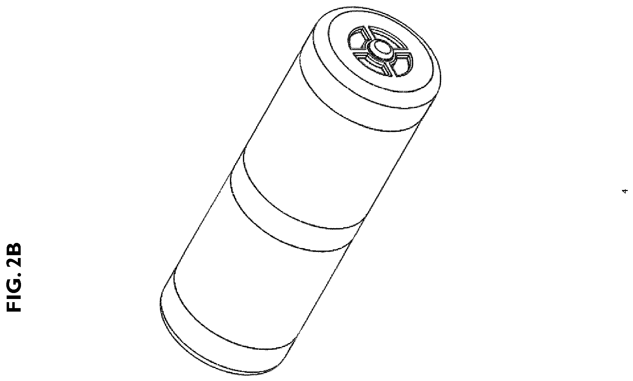

Now, it is noted that Fig. 2B depicts the illustration of Fig. 2A in a fully assembled manner and in an inactivated state.

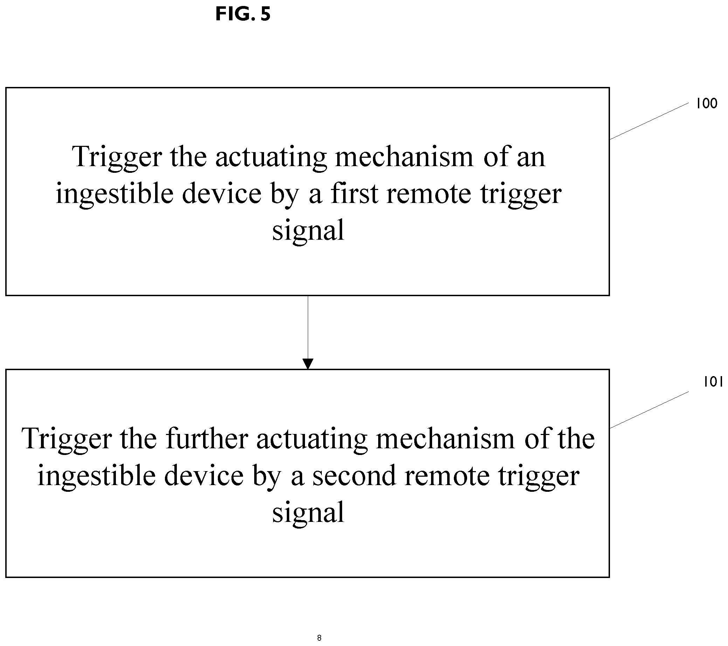

Before the final Fig. 5 showing an exemplary embodiment of the second aspect of the invention will be explained, the first aspect of the invention and its advantages are summarized in the following. The exemplary embodiment of the inventive ingestible device 10 described above can be seen as a remotely activatable dual-sampling pill, which especially consists of only a few elements. Thus, manufacturing such an ingestible device is advantageously very cheap.

The toroidal reservoir 2 and the further toroidal reservoir 4 is strong enough to withstand some pressure. Therefore, it can function as a strut to keep the outer shell of the ingestible device 10 fixed in the inactivated state. Due to its puncturable nature, it can function as part of the actuating mechanism. Also, the toroidal reservoir 2 and the further toroidal reservoir 4 can be filled with a drug containing liquid resulting a dual functionality for controlled sampling actuation and drug delivery. In particular, the toroidal reservoir 2 is used for storing and delivering the drug and for decompressing the string in a controlled way so as to control the sampling of the material. In addition, the toroidal reservoir 2 and the further toroidal reservoir 4 contains fewer complex mechanical parts and a simpler design compared to US 2021/0345904 A1. Therefore, costs of manufacturing and assembly can be much lower. Also, the reliability of the actuation mechanism can be much better.

The cap section, such as the first and second body parts 28, 31 or the first and second outer shells 27, 32, respectively, of Fig. 2A, slides over the electronics module such as the electronics module of Fig. 3. The cap lid, such as the screwable cap 23 and the further screwable cap 24 of Fig. 2A, can be screwed on the cap section. Furthermore, the cap lid contains a passive umbrella valve such as the umbrella valve 21 and the further umbrella valve 22 according to Fig. 2A. This passive umbrella valve enables one-directional flow, enabling sampling and closing of the top of the sample chamber such as the first chamber 11 and the third chamber 13 of Fig. 1A. The bottom of the sample chamber is sealed off by an O-ring, such as the O-rings 26a, 26b according to Fig. 1A or Fig. 2A, respectively, that is placed just below the top ridge of the electronics module.

Advantageously, the screwable cap lid enables easy access to the sample chamber. This can be very helpful for effortless injecting a quencher as well as simple sample removal. The overall size of the remotely activatable dual-sampling pill in the inactivated state is not exceeding 24 millimeters in length and/or 9 millimeters in diameter, which advantageously makes the pill relatively easy to swallow. Advantageously, the hollow electronics module or the hollow body portion, respectively, has enough space inside to implement the power/energy source, the sensors, and further electronics such as communication, preferably wireless communication, and at least one antenna.

Moreover, as a further advantage, the middle section of the body portion, such as the body portion 25 of Fig. 1A, is directly exposed to the gastrointestinal (GI) fluid. Accordingly, sensors implemented in this area of the pill can therefore measure GI fluid in real time. These measurements contribute the place determination of the pill and give insight in patient health or animal health, respectively.

Further advantageously, the power source or energy source, respectively, can generate more than enough current to activate the actuating mechanism. The actuating mechanism especially causes heating of the puncturing element or physical movement of the puncturing element. Especially due to this heating or movement of the puncturing element, the toroidal reservoir is punctured or deflated, allowing a vertical or lateral movement of the compression spring such as the spring 15 or the further spring 17.

The compression spring is located in between the cap section and the top ridge of the electronics module. This lateral movement enlarges the volume of the sample chamber, creating an under pressure that will be released by taking in GI fluid from the GI tract at the desired location. Simultaneously, the lateral movement also leads to a volume reduction in the drug container, such as the second chamber 12 and the fourth chamber 14 of Fig. 2A, medial to the spring, enabling liquid or drug release at the desired location.

As it can generally be seen, the remotely activatable dual-sampling pill especially consists out of three major parts that slide over each other, allowing for an expansion of the device.

Once, the device reaches the activated state, it hovers around dimensions not exceeding 24 millimeters in length and/or 9 millimeters in diameter. As already mentioned above, the remotely activatable dual-sampling pill comprises only few elements and most or even all of them are very easy to fabricate, for example via 3D printing among other methods. The sliding mechanism is a reliable way to achieve sampling, while diminishing the chance of failure. Moreover, the remotely activatable dual-sampling pill in its entirety preferably is nonmagnetic, making it MRI (magnetic resonance imaging) compatible.

Further advantageously, the ingestible device has two actuating mechanisms, one for each sampling chamber. Each of these mechanisms may especially comprise a puncturing element that after heating or moving deflates the toroidal reservoir. As the toroidal reservoir is deflated, the spring decompresses which in-turn enables the capsule module to freely move in a lateral direction. The lateral movement is achieved by decompression of the spring.

It is particularly advantageous that this actuating mechanism requires very little current as well as a sufficiently low voltage, and therefore avoids having to make use of large batteries making it extremely suitable for the remotely activatable dual-sampling pill. It is further noted again that sampling and delivery are enabled by the previously discussed lateral movement of the capsule caused by the release of the compression spring. The lateral movement of the capsule induces an expansion of the sample chamber forming an under pressure inside the sample chamber that can only be released by taking in a sample. Synchronously, the lateral movement of the capsule also causes a volume reduction in the drug container medial to the spring, creating an over pressure that can only be discharged by drug release.

As already discussed above, the inventive ingestible device advantageously provides closed off compartments because the sample chambers and drug containers are sealed off by an umbrella valve on one end and by an O-ring on the other end. Further advantageously, both components are very cheap and easily obtainable. As a further advantage, the inventive ingestible device allows for an easy sample removability because the capsule modules have a screwable cap that allows for direct sample removal. Accordingly, the chance of sample spillage is made non-existent.

Furthermore, as already discussed above, the ingestible device according to the invention provides possible space for sensors because the electronics module of the remotely activatable dual-sampling pill is large enough to contain sensors. The sensors can advantageously determine time and position to enable sampling at the correct location in the GI tract. Moreover, this also allows for continuous measurements of GI fluid.

The major advantages of the remotely activatable dual-sampling pill or the inventive ingestible device, respectively, can be summarized in a nutshell as follows:
1. The device allows for noninvasive active gastrointestinal fluid sampling.
2. The device is simple to fabricate exemplarily via 3D printing. Other parts are easily obtainable.
3. The screwable cap lids enable effortless sample retrieval.
4. The spacious interior of the electronics module leaves plenty of room for the energy source, electronics, and sensors. The implemented sensors that measure GI fluid in real time contribute to the place determination of the pill and give insight in patient health.
5. Due to overall pill size, in the inactivated state, not exceeding the already discussed size, the administration of the pill is comfortable for the user.
6. The remotely activatable dual-sampling pill is MRI compatible.
7. The remotely activatable dual-sampling pill comprises a build in localization component.
8. There is no sample contamination due to closed off sample chambers by passive umbrella valves and O-rings.
9. The remotely activatable dual-sampling pill is very easy to assemble.
10. Due to overall pill size, in the activated state, not exceeding the size as discussed above, the chance of pill retention is strongly reduced.
11. In addition to sampling, the remotely activatable dual-sampling pill can deliver drug into the GI tract (for instance, drug delivery). The same actuation mechanism is used for sampling and simultaneous delivery.

Finally, Fig. 5 shows a flow chart of an embodiment of the inventive method for using an ingestible device according to the first aspect of the invention such as the ingestible device 10 as explained above. In step 100, the actuating mechanism of the ingestible device is triggered by a first remote trigger signal. The first remote trigger signal may be a trigger signal transmitted from the external device 57 to the remote trigger unit 54 as depicted in FIG. 4. In addition to this or as an alternative, in step 101, the further actuating mechanism of the ingestible device is triggered by transmitting a second remote trigger signal. The second remote trigger signal may be a trigger signal transmitted from the external device 57 to the remote trigger unit 54 as depicted in FIG. 4. The second remote trigger signal is used to activate the further actuating mechanism so that the material is collected in the third chamber and the liquid stored in the fourth chamber is released to the environment of the ingestible device 10.

While various embodiments of the present invention have been described above, it should be understood that they have been presented by way of example only, and not limitation. Numerous changes to the disclosed embodiments can be made in accordance with the disclosure herein without departing from the scope of the invention. Thus, the breadth and scope of the present invention should not be limited by any of the above described embodiments. Rather, the scope of the invention should be defined in accordance with the following claims.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An ingestible device (10) for sampling material in a gastrointestinal (GI) tract, the ingestible device (10) comprising:
a first chamber (11) being enlargeable in space, comprising an inlet (6), and configured to be filled with a material to be sampled;
a second chamber (12) comprising a toroidal reservoir (2) being diminishable in space, configured to be filled with liquid to be delivered; and
an actuating mechanism comprising:
a spring (15) being compressed if the actuating mechanism has not been triggered yet,
a puncturing element (58) operatively coupled to the toroidal reservoir (2), and wherein the puncturing element (58) is configured to deflate the toroidal
reservoir (2) if the actuating mechanism is triggered, wherein the spring (15) is arranged with respect to the toroidal reservoir (2) such that deflating the toroidal reservoir (2) leads to a decompression of the spring (15), wherein the spring (15) is arranged with respect to the first chamber (11) such that the first chamber (11) is enlarged in the case of the decompression of the spring (15), wherein the first chamber (11) enlarges to collect the material to be sampled through the inlet (6), and wherein the spring (15) is arranged with respect to the second chamber (12) such that the second chamber (12) is diminished in the case of the decompression of the spring (15), wherein the second chamber (12) diminishes to release the liquid through at least one outlet of the toroidal reservoir.

2. The ingestible device (10) according to claim 1,
wherein the first chamber (11) collects the material through the inlet (6) due to an under pressure, wherein the inlet (6) is especially sealed when the material is collected, and/or
wherein the toroidal reservoir (2) in the second chamber (12) contains the liquid to be released through the at least one outlet due to an over pressure when the second chamber (12) is diminished.

3. The ingestible device (10) according to claims 1 or 2, further comprises:
a third chamber (13) being enlargeable in space, comprising a further inlet (8), and to be filled with further material to be sampled,
a fourth chamber (14) comprising a further toroidal reservoir (4) being diminishable in space, comprising at least one further outlet, and containing further liquid to be released, and
a further actuating mechanism,
wherein the further actuating mechanism is configured such that triggering the further actuating mechanism leads to deflate the further toroidal reservoir (4) so that the third chamber (13) enlarges to collect the further material through the further inlet (8) due to an under pressure, and the fourth chamber (14) diminishes to release the further liquid through the at least one further outlet due to an over pressure.

4. The ingestible device (10) according to any of the preceding claims,
wherein the toroidal reservoir (2, 4) comprises:
a base part (40,41) having an opening;
an umbrella valve (42,43) being pressed in the opening of the base part (40,41); and
a stretchable sheet (44,45) positioned on top of the base part (40,41), wherein the stretchable sheet (44,45) is configured to inflate when filled with the liquid through the umbrella valve (42,43), wherein the stretchable sheet (44,45) is configured to deflate when the stretchable sheet (44,45) is punctured.

5. The ingestible device (10) according to claim 4, wherein the toroidal reservoir (2,4) is filled with the liquid through the umbrella valve (42,43) coupled to the inlet of the second chamber (12) or the fourth chamber (14), wherein the umbrella valve (42,43) prevents the filled in liquid from flowing out of the toroidal reservoir (2) if the actuating mechanism has not been triggered yet.

6. The ingestible device (10) according to claim 4, wherein an inner diameter of the base part (40,41) is in a range from 3 millimeters to 7 millimeters and an outer diameter of the base part (40,41) is in a range from 7 millimeters to 12 millimeters, and/or wherein a diameter of the umbrella valve (42,43) is in a range from 2.5 millimeters to 6.5 millimeters, and/or a height of the toroidal reservoir (2) when deflated is in a range from 2 millimeters to 3 millimeters and a height of the toroidal reservoir (2) when inflated is in a range from 4 millimeters to 6 millimeters.

7. The ingestible device (10) according to any of the claims 3 to 6,
wherein the further actuating mechanism comprises:
a further spring (17) being compressed if the further actuating mechanism has not been triggered yet,
a further puncturing element operatively coupled to the further toroidal reservoir (4), and wherein the further puncturing element is configured to deflate the further toroidal reservoir (4) if the further actuating mechanism is triggered,
wherein the further spring (17) is arranged with respect to the further toroidal reservoir (4) such that deflating the further toroidal reservoir (4) leads to a decompression of the further spring (17),
wherein the further spring (17) is arranged with respect to the third chamber (13) such that the third chamber (13) is enlarged in the case of the decompression of the further spring (17), and
wherein the further spring (17) is arranged with respect to the fourth chamber (14) such that the fourth chamber (14) is diminished in the case of the decompression of the further spring (17).

8. The ingestible device (10) according to any of the preceding claims,
wherein the inlet (6) comprises a valve, preferably a passive valve, more preferably a passive one-way valve, most preferably an umbrella valve.

9. The ingestible device (10) according to any of preceding claims,
wherein the first chamber (11) comprises a removable portion, preferably a screwable cap (23), for access to the internal space of the first chamber (11)

10. The ingestible device (10) according to any of the preceding claims,
wherein the material comprises or is gastrointestinal content, and/or
wherein the liquid comprises or is a drug.

11. The ingestible device (10) according to any of the preceding claims,
wherein at least a part of the inner space of the first chamber (11) comprises a stabilizing substance, preferably a quencher, especially for preventing the material from additional chemical reactions.

12. The ingestible device (10) according to any of the preceding claims,
wherein the ingestible device further comprises a body portion (25) especially providing a hollow inner space being preferably sealed against the environment of the body portion (25) exemplarily with the aid of at least one sealing, especially in the form of an O-ring, and
wherein the first chamber (11) and the second chamber (12) are attached to the body portion (25).

13. The ingestible device (10) according to claim 12, wherein the body portion (25), especially the hollow inner space of the body portion, comprises at least one of:
an energy source (52) for supplying electric power to the actuating mechanism,
a remote trigger unit (54), being especially supplied the electric power by the energy source (52), and configured to receive at least one remote trigger signal in order to trigger the actuating mechanism,
a data recording unit (55) for recording data, preferably environment data and/or localization data, during usage of the ingestible device (10),
a wireless data transmission unit (56) for wirelessly transmitting data, preferably environment data and/or localization data, more preferably environment data and/or localization data in real time, during usage of the ingestible device (10), and/or
wherein the body portion (25), especially not the hollow inner space of the body portion (25), comprises at least one sensor, preferably a pH-value sensor.

14. The ingestible device (10) according to any of the preceding claims,
wherein the ingestible device (10) has the shape of a pill or a cylinder, and/or
wherein the length of the ingestible device (10) is lower than 24 millimeters, especially 23 millimeters, preferably after the actuating mechanism has been triggered, and/or
wherein the diameter of the ingestible device is lower than 9 millimeters, especially 8 millimeters.

## Patentansprüche

1. Einnehmbare Vorrichtung (10) zum Entnehmen von Materialproben im Magen-Darm-Trakt (GI), wobei die einnehmbare Vorrichtung (10) Folgendes umfasst:
eine erste Kammer (11), die räumlich vergrößert werden kann, einen Einlass (6) umfasst und so konfiguriert ist, dass sie mit einem zu beprobenden Material gefüllt werden kann;
eine zweite Kammer (12), die einen torischen Behälter (2) umfasst, dessen Raum verkleinert werden kann und der so konfiguriert ist, dass er mit der abzugebenden Flüssigkeit gefüllt werden kann; und
einen Betätigungsmechanismus, umfassend:
eine Feder (15), die komprimiert ist, wenn der Betätigungsmechanismus noch nicht ausgelöst wurde,
ein Durchstechelement (58), das operativ mit dem torischen Behälter (2) gekoppelt ist, und wobei das Durchstechelement (58) so konfiguriert ist, dass es den torischen Behälter (2) entleert, wenn der Betätigungsmechanismus ausgelöst wird,
wobei die Feder (15) in Bezug auf den torischen Behälter (2) so angeordnet ist, dass Entleeren des torischen Behälters (2) zu einer Dekompression der Feder (15) führt, wobei die Feder (15) in Bezug auf die erste Kammer (11) so angeordnet ist, dass die erste Kammer (11) im Falle der Dekompression der Feder (15) vergrößert wird, wobei sich die erste Kammer (11) vergrößert um das zu beprobende Material durch den Einlass (6) zu sammeln, und
wobei die Feder (15) in Bezug auf die zweite Kammer (12) so angeordnet ist, dass die zweite Kammer (12) im Falle der Dekompression der Feder (15) verkleinert wird, wobei sich die zweite Kammer (12) verkleinert, um die Flüssigkeit durch mindestens einen Auslass des torischen Behälters freizugeben.

2. Einnehmbare Vorrichtung (10) nach Anspruch 1,
wobei die erste Kammer (11) das Material durch den Einlass (6) infolge eines Unterdrucks sammelt, wobei der Einlass (6) insbesondere abgedichtet ist, wenn das Material gesammelt wird,
und/oder
wobei der torische Behälter (2) in der zweiten Kammer (12) die Flüssigkeit enthält, die infolge eines Überdrucks durch den mindestens einen Auslass freigegeben werden soll, wenn die zweite Kammer (12) verkleinert wird.

3. Einnehmbare Vorrichtung (10) nach den Ansprüchen 1 oder 2, weiter umfassend:
eine dritte Kammer (13), die räumlich vergrößert werden kann, einen weiteren Einlass (8) umfasst und mit weiterem zu beprobendem Material gefüllt werden kann,
eine vierte Kammer (14), die einen weiteren torischen Behälter (4) umfasst, der räumlich verkleinert werden kann, mindestens einen weiteren Auslass umfasst und weitere freizugebende Flüssigkeit enthält, und
einen weiteren Betätigungsmechanismus,
wobei der weitere Betätigungsmechanismus so konfiguriert ist, dass Auslösen des weiteren Betätigungsmechanismus dazu führt, dass der weitere torische Behälter (4) entleert wird, sodass sich die dritte Kammer (13) vergrößert um das weitere Material infolge eines Unterdrucks durch den weiteren Einlass (8) zu sammeln, und sich die vierte Kammer (14) verkleinert, um die weitere Flüssigkeit infolge eines Überdrucks durch den mindestens einen weiteren Auslass freizugeben.

4. Einnehmbare Vorrichtung (10) nach einem der vorstehenden Ansprüche,
wobei der torische Behälter (2, 4) Folgendes umfasst:
ein Basisteil (40, 41), das eine Öffnung aufweist;
ein Schirmventil (42, 43), das in die Öffnung des Basisteils (40, 41) eingepresst ist; und
eine dehnbare Folie (44, 45), die oben auf dem Basisteil (40, 41) positioniert ist, wobei die dehnbare Folie (44, 45) so konfiguriert ist, dass sie sich aufbläht, wenn sie durch das Schirmventil (42, 43) mit der Flüssigkeit gefüllt wird, wobei die dehnbare Folie (44, 45) so konfiguriert ist, dass sie sich entleert, wenn die dehnbare Folie (44, 45) durchstochen wird.

5. Einnehmbare Vorrichtung (10) nach Anspruch 4, wobei der torische Behälter (2,4) durch das mit dem Einlass der zweiten Kammer (12) oder der vierten Kammer (14) verbundene Schirmventil (42,43) mit der Flüssigkeit gefüllt wird, wobei das Schirmventil (42,43) verhindert, dass die eingefüllte Flüssigkeit aus dem torischen Behälter (2) herausfließt, wenn der Betätigungsmechanismus noch nicht ausgelöst wurde.

6. Einnehmbare Vorrichtung (10) nach Anspruch 4, wobei ein Innendurchmesser des Basisteils (40, 41) in einem Bereich von 3 Millimetern bis 7 Millimetern liegt und ein Außendurchmesser des Basisteils (40, 41) in einem Bereich von 7 Millimetern bis 12 Millimetern liegt und/oder wobei ein Durchmesser des Schirmventils (42, 43) in einem Bereich von 2,5 Millimetern bis 6,5 Millimetern liegt und/oder eine Höhe des torischen Behälters (2) im entleerten Zustand in einem Bereich von 2 Millimetern bis 3 Millimetern liegt und eine Höhe des torischen Behälters (2) im aufgeblasenen Zustand in einem Bereich von 4 Millimetern bis 6 Millimetern liegt.

7. Einnehmbare Vorrichtung (10) nach einem der Ansprüche 3 bis 6,
wobei der weitere Betätigungsmechanismus Folgendes umfasst:
eine weitere Feder (17), die komprimiert ist, wenn der weitere Betätigungsmechanismus noch nicht ausgelöst wurde,
ein weiteres Durchstechelement, das operativ mit dem weiteren torischen Behälter (4) gekoppelt ist, und wobei das weitere Durchstechelement so konfiguriert ist, dass es den weiteren torischen Behälter (4) entleert, wenn der weitere Betätigungsmechanismus ausgelöst wird,
wobei die weitere Feder (17) in Bezug auf den weiteren torischen Behälter (4) so angeordnet ist, dass Entleeren des weiteren torischen Behälters (4) zu einer Dekompression der weiteren Feder (17) führt,
wobei die weitere Feder (17) in Bezug auf die dritte Kammer (13) so angeordnet ist, dass die dritte Kammer (13) im Falle der Dekompression der weiteren Feder (17) vergrößert wird, und
wobei die weitere Feder (17) in Bezug auf die vierte Kammer (14) so angeordnet ist, dass die vierte Kammer (14) im Falle der Dekompression der weiteren Feder (17) verkleinert wird.

8. Einnehmbare Vorrichtung (10) nach einem der vorstehenden Ansprüche,
wobei der Einlass (6) ein Ventil umfasst, vorzugsweise ein passives Ventil, bevorzugter ein passives Einwegventil, besonders bevorzugt ein Schirmventil.

9. Einnehmbare Vorrichtung (10) nach einem der vorstehenden Ansprüche,
wobei die erste Kammer (11) einen abnehmbaren Abschnitt, vorzugsweise eine schraubbare Kappe (23) für Zugang zum Innenraum der ersten Kammer (11) umfasst.

10. Einnehmbare Vorrichtung (10) nach einem der vorstehenden Ansprüche,
wobei das Material Magen-Darm-Inhalt umfasst oder ist, und/oder wobei die Flüssigkeit ein Arzneimittel umfasst oder ein Arzneimittel ist.

11. Einnehmbare Vorrichtung (10) nach einem der vorstehenden Ansprüche,
wobei zumindest ein Teil des Innenraums der ersten Kammer (11) eine stabilisierende Substanz, vorzugsweise einen Quencher umfasst, insbesondere um weitere chemische Reaktionen des Materials zu verhindern.

12. Einnehmbare Vorrichtung (10) nach einem der vorstehenden Ansprüche,
wobei die einnehmbare Vorrichtung weiter einen Körperabschnitt (25) umfasst, der insbesondere einen hohlen Innenraum bereitstellt, der vorzugsweise gegenüber der Umgebung des Körperabschnitts (25) abgedichtet ist, beispielsweise mit Hilfe mindestens einer Dichtung, insbesondere in Form eines O-Rings, und wobei die erste Kammer (11) und die zweite Kammer (12) an dem Körperabschnitt (25) befestigt sind.

13. Einnehmbare Vorrichtung (10) nach Anspruch 12,
wobei der Körperabschnitt (25), insbesondere der hohle Innenraum des Körperabschnitts, mindestens eines umfasst von:
einer Energiequelle (52) zur Versorgung des Betätigungsmechanismus mit elektrischer Energie,
einer Fernauslöseeinheit (54), die insbesondere von der Energiequelle (52) mit elektrischer Energie versorgt wird und so konfiguriert ist, dass sie mindestens ein Fernauslösesignal empfängt, um den Betätigungsmechanismus auszulösen,
einer Datenaufzeichnungseinheit (55) zum Aufzeichnen von Daten, vorzugsweise Umgebungsdaten und/oder Lokalisierungsdaten während Verwendung der einnehmbaren Vorrichtung (10),
einer drahtlosen Datenübertragungseinheit (56) zur drahtlosen Übertragung von Daten, vorzugsweise Umgebungsdaten und/oder Lokalisierungsdaten, bevorzugter Umgebungsdaten und/oder Lokalisierungsdaten in Echtzeit während Verwendung der einnehmbaren Vorrichtung (10), und/oder wobei der Körperabschnitt (25), insbesondere nicht der hohle Innenraum des Körperabschnitts (25), mindestens einen Sensor umfasst, vorzugsweise einen pH-Wert-Sensor.

14. Einnehmbare Vorrichtung (10) nach einem der vorstehenden Ansprüche,
wobei die einnehmbare Vorrichtung (10) die Form einer Pille oder eines Zylinders aufweist, und/oder
wobei die Länge der einnehmbaren Vorrichtung (10) weniger als 24 Millimeter, insbesondere 23 Millimeter, vorzugsweise nachdem der Betätigungsmechanismus ausgelöst wurde, beträgt, und/oder wobei der Durchmesser der einnehmbaren Vorrichtung kleiner als 9 Millimeter, insbesondere 8 Millimeter ist.

## Revendications

1. Dispositif ingérable (10) pour échantillonner une matière dans un tractus gastro-intestinal (GI), le dispositif ingérable (10) comprenant :
une première chambre (11) pouvant être agrandie dans l'espace, comprenant une entrée (6), et configurée pour être remplie d'une matière à échantillonner;
une deuxième chambre (12) comprenant un réservoir toroïdal (2) pouvant être diminué dans l'espace, configuré pour être rempli d'un liquide à délivrer ; et
un mécanisme d'actionnement comprenant :
un ressort (15) qui est comprimé si le mécanisme d'actionnement n'a pas encore été déclenché,
un élément de perforation (58) couplé de manière fonctionnelle au réservoir toroïdal (2), et dans lequel l'élément de perforation (58) est configuré pour dégonfler le réservoir toroïdal (2) si le mécanisme d'actionnement est déclenché,
dans lequel le ressort (15) est agencé par rapport au réservoir toroïdal (2) de telle sorte qu'un dégonflage du réservoir toroïdal (2) entraîne une décompression du ressort (15), dans lequel le ressort (15) est agencé par rapport à la première chambre (11) de telle sorte que la première chambre (11) soit agrandie en cas de décompression du ressort (15), dans lequel la première chambre (11) s'agrandit pour collecter la matière à échantillonner via l'entrée (6), et
dans lequel le ressort (15) est agencé par rapport à la deuxième chambre (12) de telle sorte que la deuxième chambre (12) soit diminuée en cas de décompression du ressort (15), dans lequel la deuxième chambre (12) diminue pour libérer le liquide via au moins une sortie du réservoir toroïdal.

2. Dispositif ingérable (10) selon la revendication 1,
dans lequel la première chambre (11) collecte la matière via l'entrée (6) en raison d'une sous-pression, dans lequel l'entrée (6) est en particulier scellée lorsque la matière est collectée,
et/ou
dans lequel le réservoir toroïdal (2) dans la deuxième chambre (12) contient le liquide à libérer via la au moins une sortie en raison d'une surpression lorsque la deuxième chambre (12) est diminuée.

3. Dispositif ingérable (10) selon les revendications 1 ou 2, comprenant en outre :
une troisième chambre (13) pouvant être agrandie dans l'espace, comprenant une entrée supplémentaire (8), et destinée à être remplie d'une matière supplémentaire à échantillonner,
une quatrième chambre (14) comprenant un réservoir toroïdal supplémentaire (4) pouvant être diminuée dans l'espace, comprenant au moins une sortie supplémentaire, et contenant du liquide supplémentaire à libérer, et
un mécanisme d'actionnement supplémentaire,
dans lequel le mécanisme d'actionnement supplémentaire est configuré de telle sorte qu'un déclenchement du mécanisme d'actionnement supplémentaire conduise à dégonfler le réservoir toroïdal supplémentaire (4) de telle sorte que la troisième chambre (13) s'agrandit pour collecter la matière supplémentaire via l'entrée supplémentaire (8) en raison d'une sous-pression, et la quatrième chambre (14) diminue pour libérer le liquide supplémentaire via la au moins une sortie supplémentaire en raison d'une surpression.

4. Dispositif ingérable (10) selon l'une quelconque des revendications précédentes,
dans lequel le réservoir toroïdal (2, 4) comprend :
une partie de base (40, 41) présentant une ouverture ;
une valve parapluie (42, 43) qui est pressée dans l'ouverture de la partie de base (40, 41) ; et
une feuille étirable (44, 45) positionnée sur le dessus de la partie de base (40, 41), dans lequel la feuille étirable (44, 45) est configurée pour se gonfler lorsqu'elle est remplie du liquide via la valve parapluie (42, 43), dans lequel la feuille étirable (44, 45) est configurée pour se dégonfler lorsque la feuille étirable (44, 45) est perforée.

5. Dispositif ingérable (10) selon la revendication 4, dans lequel le réservoir toroïdal (2, 4) est rempli du liquide via la valve parapluie (42, 43) couplée à l'entrée de la deuxième chambre (12) ou de la quatrième chambre (14), dans lequel la valve parapluie (42, 43) empêche le liquide versé de s'écouler hors du réservoir toroïdal (2) si le mécanisme d'actionnement n'a pas encore été déclenché.

6. Dispositif ingérable (10) selon la revendication 4, dans lequel un diamètre intérieur de la partie de base (40, 41) est dans une plage allant de 3 millimètres à 7 millimètres et un diamètre extérieur de la partie de base (40, 41) est dans une plage allant de 7 millimètres à 12 millimètres, et/ou dans lequel un diamètre de la valve parapluie (42, 43) est dans une plage allant de 2,5 millimètres à 6,5 millimètres, et/ou une hauteur du réservoir toroïdal (2) lorsqu'il est dégonflé est dans une plage allant de 2 millimètres à 3 millimètres et une hauteur du réservoir toroïdal (2) lorsqu'il est gonflé est dans une plage allant de 4 millimètres à 6 millimètres.

7. Dispositif ingérable (10) selon l'une quelconque des revendications 3 à 6,
dans lequel le mécanisme d'actionnement supplémentaire comprend :
un ressort supplémentaire (17) qui est comprimé si le mécanisme d'actionnement supplémentaire n'a pas encore été déclenché,
un élément de perforation supplémentaire couplé de manière fonctionnelle au réservoir toroïdal supplémentaire (4), et dans lequel l'élément de perforation supplémentaire est configuré pour dégonfler le réservoir toroïdal supplémentaire (4) si le mécanisme d'actionnement supplémentaire est déclenché,
dans lequel le ressort supplémentaire (17) est agencé par rapport au réservoir toroïdal supplémentaire (4) de telle sorte qu'un dégonflage du réservoir toroïdal supplémentaire (4) entraîne une décompression du ressort supplémentaire (17),
dans lequel le ressort supplémentaire (17) est agencé par rapport à la troisième chambre (13) de telle sorte que la troisième chambre (13) soit agrandie en cas de décompression du ressort supplémentaire (17), et
dans lequel le ressort supplémentaire (17) est agencé par rapport à la quatrième chambre (14) de telle sorte que la quatrième chambre (14) soit diminuée en cas de décompression du ressort supplémentaire (17).

8. Dispositif ingérable (10) selon l'une quelconque des revendications précédentes,
dans lequel l'entrée (6) comprend une vanne, de préférence une valve passive, plus préférentiellement une valve unidirectionnelle passive, le plus préférentiellement une valve parapluie.

9. Dispositif ingérable (10) selon l'une quelconque des revendications précédentes,
dans lequel la première chambre (11) comprend une partie amovible, de préférence un bouchon vissable (23), pour accéder à l'espace interne de la première chambre (11).

10. Dispositif ingérable (10) selon l'une quelconque des revendications précédentes,
dans lequel la matière comprend ou est un contenu gastro-intestinal, et/ou dans lequel le liquide comprend ou est un médicament.

11. Dispositif ingérable (10) selon l'une quelconque des revendications précédentes,
dans lequel au moins une partie de l'espace intérieur de la première chambre (11) comprend une substance stabilisante, de préférence un désactivateur, en particulier pour empêcher la matière de subir des réactions chimiques supplémentaires.

12. Dispositif ingérable (10) selon l'une quelconque des revendications précédentes,
dans lequel le dispositif ingérable comprend en outre une partie de corps (25) fournissant en particulier un espace intérieur creux qui est de préférence scellé vis-à-vis de l'environnement de la partie de corps (25) par exemple à l'aide d'au moins un joint d'étanchéité, en particulier sous la forme d'un joint torique, et dans lequel la première chambre (11) et la deuxième chambre (12) sont fixées à la partie de corps (25).

13. Dispositif ingérable (10) selon la revendication 12,
dans lequel la partie de corps (25), en particulier l'espace intérieur creux de la partie de corps, comprend au moins une parmi :
une source d'énergie (52) pour alimenter en énergie électrique le mécanisme d'actionnement,
une unité de déclenchement à distance (54), alimentée en particulier en énergie électrique par la source d'énergie (52), et configurée pour recevoir au moins un signal de déclenchement à distance afin de déclencher le mécanisme d'actionnement,
une unité d'enregistrement de données (55) pour enregistrer des données, de préférence des données environnementales et/ou des données de localisation, lors de l'utilisation du dispositif ingérable (10),
une unité de transmission de données sans fil (56) pour transmettre des données sans fil, de préférence des données environnementales et/ou des données de localisation, plus préférentiellement des données environnementales et/ou des données de localisation en temps réel, lors de l'utilisation du dispositif ingérable (10), et/ou dans lequel la partie de corps (25), en particulier pas l'espace intérieur creux de la partie de corps (25), comprend au moins un capteur, de préférence un capteur de valeur de pH.

14. Dispositif ingérable (10) selon l'une quelconque des revendications précédentes,
dans lequel le dispositif ingérable (10) présente la forme d'une pilule ou d'un cylindre, et/ou
dans lequel la longueur du dispositif ingérable (10) est inférieure à 24 millimètres, en particulier 23 millimètres, de préférence après le déclenchement du mécanisme d'actionnement, et/ou dans lequel le diamètre du dispositif ingérable est inférieur à 9 millimètres, en particulier 8 millimètres.
